Europäisches Patentamt

European Patent Office

Office européen des brevets

(19)

(11) Publication number : **0 541 347 A2**

(12) # EUROPEAN PATENT APPLICATION

(21) Application number : 92310092.9

(22) Date of filing : 04.11.92

(51) Int. Cl.$^5$ : **A61K 7/02, A61K 7/48, A61K 7/06**

(30) Priority : 07.11.91 GB 9123649
04.09.92 GB 9218791

(43) Date of publication of application :
12.05.93 Bulletin 93/19

(84) Designated Contracting States :
AT BE CH DE DK ES FR GB GR IE IT LI NL PT
SE

(71) Applicant : UNILEVER PLC
Unilever House Blackfriars
London EC4P 4BQ (GB)
(84) GB IE

(71) Applicant : UNILEVER N.V.
Weena 455
NL-3013 AL Rotterdam (NL)
(84) BE CH DE DK ES FR GR IT LI NL PT SE AT

(72) Inventor : Haq, Zia
32 Latchford Road
Gayton, Wirral, Merseyside L60 3RW (GB)
Inventor : Lyle, Ian Gardner
60 Highland Avenue, Aston Park
Deeside, Clwyd CH5 1XQ, Wales (GB)

(74) Representative : Ford, Michael Frederick et al
MEWBURN ELLIS 2 Cursitor Street
London EC4A 1BQ (GB)

(54) Cosmetic composition.

(57)  A cosmetic composition suitable for use as a make-up remover or as a degreasing composition for hair or skin, comprises an aqueous solution of from 1 to 25% by weight of one or more non-ionic secondary branched or straight chain, or primary branched chain, alcohol alkoxylate surfactants having an average HLB value of 10 to 15. The composition preferably has a cloud point in the range 10 to 40°C, and in preferred embodiments also includes an anionic surfactant, electrolyte and thickener.

EP 0 541 347 A2

This invention relates to cosmetic compositions, in particular aqueous, surfactant-containing cosmetic compositions. More specifically, the invention relates to such cosmetic compositions which are suitable for use as make-up removers and as degreasing compositions for hair and skin.

To provide a cosmetic composition effective as a make-up remover or a degreasing product, it is desirable that the composition has a high efficacy in terms of its ability to remove greasy material, especially greasy make-up compositions such as lipstick, eye shadow or foundation. Additionally, the composition should have a high degree of ease of rinsing, in that it should be taken up rapidly by water when rinsing, and the composition should also have a low degree of irritancy (i.e. be mild), not only with regard to the skin, but in particular with regard to the eyes (as is required, for example, for the removal of eye make-up).

Degreasing compositions, e.g. for the removal of heavy duty oils and greases from the hands, are known, for example from EP-A-0002334. The disclosed compositions comprise certain non-ionic surfactants which are alkylene oxide condensates of long chain linear alcohols, phenols or acids (having specifically 9 ethylene oxide/propylene oxide groups per molecule), which have a preferred HLB value of 12.2-12.5.

Aqueous surfactant compositions containing secondary alcohol ethoxylates are also known. For example, JP-A-61-73799 discloses a detergent composition for cleaning a powder puff comprising a secondary C12-C14 alcohol ethoxylate containing 4-6 ethylene oxide groups per molecule. The surfactant has an HLB value of 8-14, and can be used by diluting it with up to 50% water.

We have now found that a cosmetic composition with good make-up remover suitability and good removal capabilites for all types of make-up and other greasy deposits found naturally on the hair or the skin, especially very greasy types of make-up such as Lipstick and eye make-up, and also excessive grease, e.g. sebum, deposits on the hair, can be provided by an aqueous solution of one or more non-ionic secondary branched or straight chain, or primary branched chain, alcohol ethoxylate surfactants having an average HLB value of 10-15.

Accordingly, in a first aspect the present invention provides an aqueous cosmetic composition suitable for use as a make-up remover or degreasing composition for hair or skin, the composition comprising in aqueous solution from 1 to 25% by weight of one or more non-ionic secondary branched or straight chain, or primary branched chain, alcohol alkoxylate surfactants having an average HLB value of 10 to 15.

Preferably, the non-ionic surfactant or surfactants in the composition have an average alkyl chain length of about C10-C16, more preferably about C12-C14. Preferred alkoxylate groups are ethylene oxide or propylene oxide residues, or even mixtures of such residues. Ethylene oxide groups are particularly preferred. Preferably the surfactants have an average of about 5-9 ethylene oxide groups, more preferably about 5-8, even more preferably about 5-7 ethylene oxide groups, per molecule.

Additionally we have identified that it is most desirable for the water based make-up removal or degreasing composition of the invention to have a "cloud point" in the region of around 10 to 40°C.

The theory of the "cloud point" of an aqueous non-ionic surfactant solution is well known, and is explained in detail in "Non-ionic surfactants", Volume 1 of the Surfactant Science Series, published by Marcel Dekker, NY 1967, chapter 17, the content of which is incorporated herein by reference.

From an aesthetic point of view, it is usually desirable that prior to use the cosmetic composition of the invention is clear, or at least as clear as possible, when the user observes it in the bottle, but for efficient cleansing purposes the composition should be used at or near its cloud point temperature. This is achieved by formulating the product, i.e. the defined aqueous non-ionic surfactant(s) preferably with one or more additional ingredients as described further below, such that the composition has a cloud point in the range of from about 10 to 40°C, more preferably from about 10 to 35°C, even more preferably from about 15 to 30°C.

Thus, according to a further aspect of the invention there is provided an aqueous make-up removal or degreasing composition comprising the above defined non-ionic surfactant(s), which composition has a cloud point of between about 10 and 40°C, more preferably between about 10 and 35°C, even more preferably between about 15 and 30°C.

In another aspect, the present invention provides a method of removing make-up and/or naturally present greasy material from the skin, including from the eyes, comprising applying thereto a cosmetic composition as defined above. The composition may thereafter be removed, with the make-up/greasy deposits, for example by rubbing off with a pad or such like, or by rinsing off with water.

In a further aspect of the invention, in which the above defined composition is used as a degreasing composition for hair, the composition is applied thereto and may thereafter be rinsed out with water. This treatment may usefully be carried out immediately prior to normal shampooing, i.e. as a degreasing pre-treatment, such as may be desirable for people with excessively greasy hair.

The present invention, and in particular preferred features and embodiments thereof, will now be described in detail, by way of example only.

According to the invention, the non-ionic surfactant used is present in aqueous solution in the composition at a concentration of from about 1 to 25%, more preferably from about 2 to 10%, by weight of the composition.

Preferably the non-ionic surfactant(s) used in the invention has/have an HLB value of 10-15, more preferably 10-13.

A particularly preferred non-ionic secondary alcohol ethoxylate surfactant has an alkyl chain length of C12-14, has an average of 7 ethylene oxide groups, and an HLB value of 12.1. Examples of this surfactant are SOFTANOL 70, obtainable from BP Chemicals Ltd. or Nippon Catalytic of Japan, or TERGITOL 15-S-7, available from Union Carbide. Another preferred non-ionic secondary alcohol ethoxylate surfactant has an alkyl chain length of C12-14, has an average of 5 ethylene oxide groups, and has an HLB value of 10.5. An example of this surfactant is SOFTANOL 50, obtainable from BP Chemicals Ltd. or Nippon Catalytic, or TERGITOL 15-S-5, available from Union Carbide.

A combination of non-ionic surfactants suitable for use in the invention is a mixture of the defined non-ionic surfactants, for example a 1:1 mixture of TERGITOL 15-S-9 and TERGITOL 15-S-3, both available from Union Carbide.

Whilst some of the above mentioned non-ionic surfactants or combinations of surfactants may be used as the sole surfactant component in the composition, it has been found that better results may be obtained if the composition additionally comprises an anionic surfactant, for example at a concentration of up to 5%, more preferably 0.1 to 3%, by weight of the composition.

Suitable anionic surfactants are the alkyl sulphates, alkyl ether sulphates, alkaryl sulphonates, alkyl succinates, alkyl sulphosuccinates, N-alkoyl sarcosinates, alkyl phosphates, alkyl ether phosphates, alkyl ether carboxylates, and α-olefin sulphonates, especially their sodium, magnesium, ammonium and mono-, di- and tri-ethanolamine salts. The alkyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule, and preferably contain 2 to 3 ethylene oxide units per molecule. Mixtures of anionic surfactants may be used if desired.

A preferred anionic surfactant is EMPICOL 0251, available from Albright & Wilson, a sodium lauryl ethoxy sulphate surfactant, with 3 ethylene oxide groups. Other preferred anionic surfactants include monoalkyl phosphates, acyl lactylate surfactants, or acyl taurate surfactants (e.g. acyl methyl taurate).

It has been found that the inclusion of an anionic surfactant in the composition may help the rinsability of the composition, i.e. how readily it is rinsed from the skin or hair with water.

Preferably, the pH of the cosmetic composition of the invention is in the range of about 5-8, most preferably around 5.5-7.

In a particularly preferred embodiment of the invention, the cosmetic composition contains electrolyte, such as sodium chloride, potassium sorbate or sodium citrate, preferably at a concentration of about 0.2-5% by weight of the composition, more preferably at a concentration of about 0.5-2.5% by weight of the composition. It has been found that by including electrolyte in the composition at these concentrations the solution comes as close as possible to balancing the osmotic potential of the eye, thereby minimising any irritational effects that the composition may have on the eyes during use, caused possibly for example by the surfactant(s) in the composition.

It is also thought that the addition of an electrolyte to the composition may make the surfactant less soluble in the composition, and this is thought to contribute to the reduction of irritation that the composition may cause to the eyes.

Thus, according to a further preferred embodiment of the invention, the cosmetic composition further comprises from about 0.2-5%, preferably 0.5-2.5% by weight of the composition, of an electrolyte.

According to another preferred embodiment of the invention, the cosmetic composition comprises a thickener.

Preferred thickeners include: the carbomers (CTFA definition), such as Carbopols (ex Goodrich) e.g. Carbopol 910, 934, 940, 941 and 1382; cationic polymers, such as Polymer JR (quaternised cellulose), or JAGUAR (quaternised guar gum); non-ionic polymers such as Klucel (hydroxypropyl cellulose) or Natrosol (hydroxyethyl cellulose); hydrophobically modified cellulose derivatives; high molecular weight polymers such as polyethylene glycol (PEG) and its esters or other derivatives (e.g. PEG distearate, or PEG dioleate); natural gums (e.g. guar gum, carageenan gum, locust bean gum, xanthan gum, tamarind seed gum); inorganic structurants such as swelling clays (e.g. Hectorites or Bentonites) and hydrotalcites.

The preferred concentration of the thickener in the composition will depend on the exact thickener chosen. For example, polymer-type thickeners, if used, will preferably be present in the composition at a concentration of from about 0.1 to 5% by weight, whereas inorganic structurants, if used, will preferably be present at a concentration of from about 2 to 30% by weight of the composition.

The actual thickeners that may be used in the composition have also been found to depend on the concentration of the surfactant in the composition. At a surfactant concentration of 5% by weight of the solution, preferred thickeners are Carbopol 1382, Natrosol Plus 430, kappa carageenan gum, Polymer JR 400, PEG 6000 distearate, Bentone 38, or aluminium magnesium hydroxy carbonate (hydrotalcite), especially Carbopol 1382.

Whilst not intending to be bound by theory, it is thought that the incorporation of a thickener in the composition may help to reduce the irritancy of the composition to the eyes during use. It has been found that cosmetic compositions having an HLB value of 10-15 and comprising 1 to 25% of a non-ionic secondary alcohol alkoxylate surfactant according to the invention, optionally in combination with an anionic surfactant, and preferably including both salt and a thickener (when required, e.g. in the light of the product form), with preferably the concentration of the surfactant being selected such that the cloud point of the solution is in the region of from about 10 to 40°C, provide a make-up removing or degreasing solution which has remarkably good properties in terms of its ability to remove greasy solids such as make-up, its wettability (and hence the ease and speed with which it may be rinsed off), and also its mildness to skin and eyes.

The cosmetic compositions in accordance with the invention may also comprise other ingredients conventionally used in make-up removal and other cosmetic compositions, including for example colouring agents, co-solvents such as ethanol (up to around 5% by weight of the composition), polyols such as sorbitol, glycerol, and butanediol, preservatives, perfumes, opacifiers, pearlescers and small amounts of emollient oils such as silicones (present at up to around 5% by weight of the composition).

In preferred embodiments of the invention, the make-up removal or degreasing compositions are in the form of simple liquid products e.g. as lotions, milks, gels or creams. Such product forms may if desired be provided for use in the form of impregnated wipes, pads or the like.

Alternatively, compositions of the invention may be provided for use in a mousse form. This allows the compositions to be made in a more viscous form than a pure liquid formulation, thus obviating the need for a thickener to be included in the composition.

When it is to be used in the form of a mousse, the liquid composition according to the invention may be packaged in a conventional pressurized container along with for example from about 3 to 8% by weight of the composition of a conventional propellant gas.

Compositions according to the invention which additionally contain silicones may be preferred, since it is thought that the presence of the silicone in the formulation improves the feel of the product on skin and may help reduce its irritation to eyes.

The invention will now be demonstrated by way of the following examples. All amounts given are in wt%, unless otherwise stated.

Example 1

| Component | % w/w of the composition |
|---|---|
| Softanol 70 | 8.75 |
| Empicol 0251 | 1.25 |
| Preservative (Nipagin M) | 0.20 |
| Sodium hydroxide solution | to pH 6 |
| Distilled water | to 100 |

4

Example 2

| Component | % w/w of the composition |
|---|---|
| Softanol 70 | 17.50 |
| Empicol 0251 | 2.50 |
| Preservative (Nipagin M) | 0.2 |
| Sodium hydroxide solution | to pH 6 |
| Distilled water | to 100 |

Example 3

| Component | % w/w of the composition |
|---|---|
| Softanol 70 | 4.375 |
| Empicol 0251 | 0.625 |
| Preservative (Nipagin M) | 0.3 |
| Sodium hydroxide solution | to pH 6 |
| Sodium chloride | 2 |
| Distilled water | to 100 |

The make-up removal compositions according to the invention were tested in comparative tests as to their make-up removal efficacy for various types of make-up, against known commercially available make-up removal compositions.

A "rinse off" method was used for comparative tests for the removal of lipstick and eye shadow from skin, and a "rub off" method was used for the removal of waterproof mascara from skin.

Test products - all ex. Rimmel International

    a. Colour Plus Lipstick - Truly Red
    b. Enhanced Eyeshadow - Aquarius X 464
    c. Special Formula Waterproof Mascara - Dark Navy

Make-up removal compositions

    1. Example 1
    2. Example 2
    3. Example 3
    4. Plenitude Creme Demaquillante Douce a l'Eau (L'Oreal)
    5. Sofina Make Clear Gel (Kao)
    6. Biore Cleansing Wash (Kao)
    7. Pond's Cold Cream Cleanser (Chesebrough Ponds)
    8. Pierre Robert New Skin Eye Make-up Remover (Elida Robert)

Panellists

The products were evaluated on panels of volunteer personnel, 18 being used on the lipstick test and 9 on each of the others. On each visit, the panellist was exposed to 3 different test product, applied to one volar forearm. Each panellist evaluated each make-up remover once, making a total of three visits to complete the test.

5

Test protocols

"Rinse off" method

A plastic template was used to define three 3cm x 2cm rectangular areas on the panellist's volar forearm. The colour of the skin within each was recorded using a Minolta Chroma Meter CR-100 to give a set of baseline readings (A). Each recorded result is the average of 6 individual measurements.

The designated areas were then coated with the test make-up, which was applied in a standardised way to ensure that approximately equal weights of the make-up were transferred. After drying, a second colour measurement was made (reading B).

A standard amount of test product (0.2ml or equivalent weight) was applied neat to the skin, a different product being used on each make-up patch. The cleanser was rubbed into the make-up for 20 seconds, using light finger pressure (gloved hand), after which the mixture was immediately rinsed off with running tap water at a fixed flow rate and temperature (35°C). In the case of the Ponds Cold Cream make-up remover, the removal of the make-up remover was performed not with the running water but with a dry cotton wool ball.

After all 3 patches had been similarly treated, the arms were dried with a hair drier and final colour measurements were made (reading C).

The percentage removal is calculated as: $\dfrac{B - C}{B - A} \times 100$

"Rub off" method

The protocol for this method is the same as that described for the "rinse off" method above, except that the time of application was increased to 30 seconds, and removal was aided by gentle rubbing with a damp cotton wool ball for 20 seconds. In the case of the Ponds Cold Cream make-up remover, a dry cotton wool ball was used instead of a wet one.

Results

| "Rinse off" method | | |
|---|---|---|
| | % make up removal | |
| Formulation | Lipstick | Eyeshadow |
| 1 | 80.9 | 96.7 |
| 2 | 80.7 | 96.7 |
| 3 | 80.3 | 97.4 |
| 4 | 43.2 | 55.9 |
| 5 | 82.4 | 83.9 |
| 6 | 86.0 | 96.8 |
| 7 | 76.5 | 81.8 |
| 8 | 28.4 | 95.2 |

| "Rub off" method | |
|---|---|
| Formulation | % mascara removal |
| 1 | 81.0 |
| 2 | 74.5 |
| 3 | 72.7 |
| 4 | 21.5 |
| 5 | 32.9 |
| 6 | 53.4 |
| 7 | 56.0 |
| 8 | 31.9 |

These results demonstrate that formulations according to the invention (i.e. Formulations 1, 2 and 3) have generally at least as good make-up removal efficacy as some of the best commercially available make-up removal formulations, and in some circumstances (e.g. waterproof mascara removal) notably better. The formulations also have good rinsability and mildness, are cheap to produce, and have a good consumer "feel" on use.

Example 4

Further tests were conducted to demonstrate the effect of cloud point on the efficacy of make-up removal using compositions in accordance with the invention. Formulations 9-14 were prepared as shown below and lipstick removal was measured using the "rinse off" arm wash protocol described above.

| Formulation No. | Wt % active ingredient | | | | | |
|---|---|---|---|---|---|---|
| | 9 | 10 | 11 | 12 | 13 | 14 |
| Softanol 70 | 4.375 | 4.375 | 4.375 | -- | 4.375 | 4.375 |
| Softanol 50 | -- | -- | -- | 4.375 | -- | -- |
| Empicol 0251[1] | 0.625 | 0.625 | 0.625 | 0.625 | -- | -- |
| Pationic 138C[2] | -- | -- | -- | -- | 0.625 | -- |
| Diapon K-SF[3] | -- | -- | -- | -- | -- | 0.625 |
| Potassium sorbate | 0.500 | 0.500 | 0.500 | 0.500 | 0.500 | 0.500 |
| Nipagin M | 0.300 | 0.300 | 0.300 | 0.300 | 0.300 | 0.300 |
| Sodium chloride | -- | 1.000 | 2.000 | -- | -- | -- |
| Disodium citrate | -- | -- | -- | 2.500 | 2.500 | 2.500 |
| Hydrochloric acid to pH | 5.3 | 5.3 | 5.3 | -- | -- | -- |
| Sodium hydroxide to pH | -- | -- | -- | 5.5 | 5.5 | 5.5 |
| Distilled Water | to 100 | to 100 | to 100 | to 100 | to 100 | to 100 |
| Cloud point (°C) | 47.0 | 28.0 | 21.5 | <0 | <0 | 32.5 |
| Lipstick removal (%) | 79.1 | 85.9 | 86.3 | 49.3 | 69.5 | 85.1 |

[1]  Sodium lauryl ethoxy sulphate, 3EO (Surfachem)

[2]  Sodium lauroyl/myristoyl lactylate (RITA Corporation)

[3]  Cocoyl N-methyl sodium taurate (Nippon Oil and Fats)

These results indicate an improved efficacy of make-up removal when the cloud point of the solution is adjusted to be close to skin temperature.

Example 5

The following composition provides a satisfactory make-up removal composition suitable for dispensing

in mousse form:

| Component | |
|---|---|
| **Phase A (Solution)** | **% weight active ingredient** |
| Softanol 70[1] | 4.375 |
| Empicol 0251[2] | 0.625 |
| Nipagin M[3] | 0.300 |
| Sobistat K[4] | 0.500 |
| Disodium hydrogen citrate | 2.500 |
| Sodium hydroxide | to pH 5.3 - 5.5 |
| Distilled water | to 100 |

| **Phase B (Propellant)** | |
|---|---|
| CAP 30[5] | 100.000 |

[1]  C12/14 secondary alcohol ethoxylate, 7EO; ex.BP.

[2]  C12/15 alkyl ether sulphate, 3EO; ex Surfachem.

[3]  Methyl parahydroxybenzoate preservative; ex Nipa Labs.

[4]  Potassium Sorbate preservative; ex Pfizer.

[5]  11% Propane, 29% Isobutane, 60% n-Butane propellant; ex Calor.

Mousse forming composition:  95% Phase A

5% Phase B.

Example 6 - Polymer thickened make-up remover with silicone.

| | Wt % active ingredient |
|---|---|
| Softanol 70 | 4.375 |
| Empicol 0251 | 0.625 |
| Nipagin M | 0.300 |
| Disodium hydrogen citrate | 2.500 |
| Potassium sorbate | 0.500 |
| Carbopol 1382 | 0.750 |
| Silicone fluid DC200/60000cs (DOW CORNING) | 5.000 |
| Perfume oil | 0.050 |
| Sodium hydroxide | to pH 5.5 |
| Distilled water | to 100 |

Example 7 - Pearlescent lotion.

|  | Wt % active ingredient |
|---|---|
| Softanol 70 | 4.375 |
| Empicol 0251 | 0.625 |
| Emalex 6300 M-ST[1] | 2.500 |
| Cutina EGMS[2] | 0.500 |
| Glucamate DOE-120[3] | 0.500 |
| Nipagin M | 0.300 |
| Potassium sorbate | 0.500 |
| Disodium hydrogen citrate | 2.500 |
| Perfume oil | 0.100 |
| Sodium hydroxide | to pH 5.5 |
| Distilled water | to 100 |

[1]    PEG-150 monostearate; ex Nihon Emulsion Co.

[2]    Ethylene glycol monostearate; ex Henkel

[3]    PEG-120 methyl glucose dioleate; ex Anstead

Example 8 - Cream

|  | Wt % active ingredient |
|---|---|
| Softanol 70 | 4.375 |
| Empicol 0251 | 0.625 |
| Xalifin 15[4] | 15.000 |
| Nipagin M | 0.300 |
| Potassium sorbate | 0.500 |
| Disodium hydrogen citrate | 2.500 |
| Perfume oil | 0.100 |
| Sodium hydroxide | to pH 5.5 |
| Distilled water | to 100 |

[4]    PEG-8 C12/14 alkyl ester, ex Vevy.

**Claims**

1. An aqueous cosmetic composition suitable for use as a make-up remover or degreasing composition for hair or skin, the composition comprising in aqueous solution from 1 to 25% by weight of one or more non-ionic secondary branched or straight chain, or primary branched chain, alcohol alkoxylate surfactants having an average HLB value of 10 to 15.

2. A cosmetic composition according to claim 1, which has a cloud point in the range 10 to 40°C.

10

3. A cosmetic composition according to claim 1 or claim 2, wherein the average alkyl chain length of the non-ionic surfactant(s) is from C10 to C16.

4. A cosmetic composition according to any one of claims 1 to 3, wherein the non-ionic surfactant(s) have an average of 5 to 9 alkylene oxide residues per molecule.

5. A cosmetic composition according to any preceding claim, wherein in the non-ionic surfactant(s) the alkylene oxide residues are residues of ethylene oxide.

6. A cosmetic composition according to any preceding claim, further comprising one or more anionic surfactants.

7. A cosmetic composition according to claim 6, wherein the one or more anionic surfactants are present in a total amount of up to 5% by weight of the composition.

8. A cosmetic composition according to any preceding claim, which has a pH in the range 5 to 8.

9. A cosmetic composition according to any preceding claim, further comprising an electrolyte.

10. A cosmetic composition according to claim 9, wherein the electrolyte is present at a concentration of from 0.2 to 5% by weight of the composition.

11. A cosmetic composition according to any preceding claim, further comprising a thickener.

12. A cosmetic composition according to claim 11, wherein the thickener is selected from the group consisting of: carbomers; cationic polymers; non-ionic polymers, especially non-ionic cellulose derivatives; high molecular weight polymers, especially polyethylene glycol and derivatives thereof; natural gums; inorganic structurants, especially swelling clays and hydrotalcites.

13. A cosmetic composition according to any preceding claim, further comprising a silicone component.

14. A cosmetic composition according to any preceding claim, which is in the form of a lotion, milk, gel or cream.

15. A cosmetic composition according to any one of claims 1 to 13, which is impregnated into a wipe or pad.

16. A cosmetic composition according to any one of claims 1 to 13, which is in the form of a mousse.

17. A method of removing make-up and/or naturally present greasy material from the skin, comprising applying thereto a cosmetic composition according to any preceding claim.

18. A method according to claim 17, further comprising the subsequent step of removing the composition from the skin, with the make-up and/or greasy deposits, by rubbing off or by rinsing off with water.

19. A method of removing greasy material from the hair, comprising applying thereto a cosmetic composition according to any one of claims 1 to 16, and subsequently rinsing with water.

20. Use as an agent for removing make-up or greasy material from hair or skin of an aqueous solution of from 1 to 25% by weight of one or more non-ionic secondary branched or straight chains, or primary branched chain, alcohol alkoxylate surfactants having an average HLB value of 10 to 15.